# FASCICULE DE BREVET EUROPEEN

(11) **EP 0 518 096 B1**
(45) Date de publication et mention de la délivrance du brevet: **27.09.1995**
(21) Numéro de dépôt: 92108573.4
(22) Date de dépôt: 21.05.1992
(51) Int. Cl.: A23C 9/123, C12N 1/20, C12N 15/01

(54) **Yogourt contenant des microorganismes vivants**
Lebendige Mikroorganismen enthaltender Joghurt
Yogurt containing living microorganisms

(30) Priorité: 14.06.1991 EP 91109807
(43) Date de publication de la demande: 16.12.1992
(73) Titulaire: SOCIETE DES PRODUITS NESTLE S.A., 1800 Vevey (CH)
(72) Inventeur: Hottinger, Herbert, CH-1807 Blonay (CH); Mignot, Olivier, CH-1807 Blonay (CH); Mollet, Beat, CH-1074 Molie-Margot (CH)

(56) Documents cités:
- EP-A- 0 081 705
- WO-A-90/05459
- US-A- 4 425 366
- US-A- 4 734 361
- PATENT ABSTRACTS OF JAPAN vol. 14, no. 220 (C-717)(4163) 10 Mai 1990
- MILCHWISSENSCHAFT vol. 43, no. 10, 1988, MüNCHEN (WESTD) pages 626 - 631; M.J. AMOROSO ET AL.: 'Glucose, galactose, fructose, lactose and sucrose utilization by lactobacillus bulgaricus and streptococcus thermophilus isolated from commercial yoghurt'
- JOURNAL OF BACTERIOLOGY vol. 172, no. 10, Octobre 1990, pages 5670 - 5676; BEAT MOLLET ET AL.: 'Spontaneous deletion formation within the beta-galactosidase gene of lactobacillus bulgaricus'
- JOURNAL OF MOLECULAR BIOLOGY vol. 98, 1975, pages 503 - 517; E.M.SOUTHERN: 'Detection of specific sequences among DNA fragments separated by gel electrophoresis'

## Description

La présente invention a pour objet un yogourt et son procédé de préparation, ainsi qu'un mutant de Lactobacillus bulgaricus et son procédé de sélection.

Le yogourt est obtenu par fermentation d'un lait avec une combinaison de souches de Streptococcus thermophilus et de Lactobacillus bulgaricus et se présente sous forme de gel contenant les souches vivantes. C'est un produit frais, à conservation limitée, même sous réfrigération. Divers procédés ont été proposés pour améliorer ses propriétés de conservation, notamment pour réduire l'augmentation de son acidité, autrement dit sa postacidification, et l'apparition d'un goût amer qui dégradent ses qualités organoleptiques.

C'est ainsi que JP-A-85256341 propose la préparation d'un yogourt doux en utilisant un levain mixte dans lequel les nombres de cellules/ml de L.bulgaricus et S.thermophilus sont dans un rapport préféré de 1:100. Si la réduction du nombre relatif de cellules de L.bulgaricus peut permettre de réduire la postacidification du yogourt en cours de conservation sous réfrigération, elle peut cependant entraîner aussi une réduction du caractère organoleptique typiquement yogourt du produit qui est dû au travail en symbiose des deux microorganismes.

US 4425366 décrit la préparation d'un yogourt longue conservation à l'aide d'une combinaison de souches de L.bulgaricus et de S.thermophilus dans laquelle la souche de L.bulgaricus présente une faible activité protéolytique. Ce document propose cependant également d'utiliser un nombre relatif de cellules de L.bulgaricus de l'ordre de 1:100.

US 4734361 décrit la sélection d'une souche de L.bulgaricus sensible aux basses températures, autrement dit présentant une faible activité à une température de conservation de 10°C tout en présentant une bonne activité à une température de fermentation de 43°C. La souche FERM BP-1041 ainsi sélectionnée a été dérivée de la souche ATCC 11842.

JP-A-90053437 décrit la préparation d'un yogourt en utilisant, en combinaison avec une souche de S.thermophilus, une souche de L.bulgaricus sélectionnée pour son incapacité (mutant artificiel SBT-0218) ou sa capacité réduite (mutant naturel SBT-0220) à fermenter le lactose. Le lait de départ doit alors être supplémenté en glucose.

WO 90/05459 propose la construction d'un L.bulgaricus sensible aux basses températures ou aux bas pH par mutation sélective sur E.coli d'un gène bêta-galactosidase prélevé sur L.bulgaricus et destiné à être réincorporé à L.bulgaricus.

La présente invention a pour but de proposer la préparation d'un yogourt dont les qualités organoleptiques ne se dégradent pas à la conservation tout en présentant un caractère traditionnel typique dû au travail en symbiose des deux microorganismes S.thermophilus et L.bulgaricus. Elle a également pour but de proposer un procédé reproductible et sûr de sélection d'un mutant de L.bulgaricus qui, utilisé en combinaison avec au moins une souche de S.thermophilus, permette de préparer un tel yogourt.

A cet effet, le yogourt selon la présente invention comprend:
- 10-20% en poids de matière sèche d'un lait animal et/ou végétal,
- de 10⁶-10¹⁰ cellules/ml d'un mutant de Lactobacillus bulgaricus dans l'ADN duquel manque un fragment comprenant une partie au moins du gène bêta-galactosidase, et
- de 10⁶-10¹⁰ cellules/ml de Streptococcus thermophilus.

De même, dans le procédé de préparation d'un yogourt selon la présente invention, on fait fermenter un lait avec une combinaison d'au moins une souche de Streptococcus thermophilus et d'un mutant de Lactobacillus bulgaricus dans l'ADN duquel manque un fragment comprenant une partie au moins du gène bêta-galactosidase.

Le mutant de Lactobacillus bulgaricus selon la présente invention présente donc un ADN où manque un fragment comprenant une partie au moins du gène bêta-galactosidase.

Enfin, dans le procédé de sélection d'un mutant de L.bulgaricus selon la présente invention,
- on réalise un criblage d'une souche mère de L.bulgaricus sur plaque X-gal pour isoler des colonies présentant une mutation bêta-galactosidase moins,
- on soumet ces colonies de mutants à un test de stabilité dans un milieu contenant du lactose comme seule source d'énergie,
- on digère l'ADN chromosomique de mutants stables à l'aide d'enzymes de restriction et on le soumet à un test de détection de fragments compris dans une région comprenant le gène bêta-galactosidase, et
- l'on sélectionne un mutant présentant un ADN dans lequel manque un fragment comprenant une partie au moins du gène bêta-galactosidase.

On a constaté en effet qu'il est ainsi possible de proposer un yogourt dans lequel la postacidification et l'apparition d'un goût amer à la conservation soient notablement réduits et dont par ailleurs les qualités organoleptiques présentent un caractère traditionnel typique du yogourt dû au travail en symbiose des deux microorganismes S.thermophilus et L.bulgaricus.

On a constaté notamment que le mutant de L.bulgaricus selon la présente invention présente la propriété surprenante de ne provoquer qu'une faible postacidification à la conservation tout en travaillant de manière active en symbiose avec S.thermophilus durant la fermentation, les nombres respectifs de cellules de ces deux microorganismes pouvant en particulier se trouver dans un rapport relativement équilibré, notamment un rapport de environ 1:20 - 1:1, aussi bien au départ de l'incubation que dans le yogourt obtenu.

Dans deux formes d'exécution préférées du yogourt, de son procédé de préparation, du mutant et de son procédé de sélection, ledit fragment d'ADN manquant soit ne comprend qu'une partie du gène bêta-galactosidase, soit comprend une partie au moins du gène bêta-galactosidase et s'étend au moins en aval de ce gène sur au moins 1,0 kb.

La première de ces formes d'exécution préférées est plus particulièrement destinée à une utilisation dans des pays à climat tempéré, voire relativement froid. La seconde de ces formes d'exécution préférées est plus particulièrement destinée à une utilisation dans des pays à climat relativement chaud.

Ladite première forme d'exécution préférée est également susceptible d'être utilisée dans des pays à climat relativement chaud pour autant qu'on y dispose de moyens de réfrigération adéquats, notamment d'une chaîne du froid. De même, ladite deuxième forme d'exécution préférée est également susceptible d'être utilisée dans des pays à climat relativement froid sans faire usage de moyens de réfrigération particuliers, en se passant notamment d'une chaîne du froid.

Dans le cadre de ladite première forme d'exécution préférée, le mutant de L.bulgaricus est apparemment incapable de fermenter seul le lactose, mais, cultivé dans un lait en symbiose avec S.thermophilus, il est capable d'acidifier ce lait pratiquement aussi bien que la souche mère dont il est issu. L'addition de faibles quantités de glucose permet de moduler la vitesse de cette acidification et le niveau de postacidification à la conservation.

Dans le cadre de ladite deuxième forme d'exécution préférée, le mutant de L.bulgaricus est apparemment incapable de fermenter seul le lactose, et, cultivé dans un lait en symbiose avec S.thermophilus, il acidifie ce lait à une vitesse moins grande et jusqu'à un pH moins élevé que la souche mère dont il est issu. L'addition d'une faible quantité de glucose permet d'accélérer un peu la vitesse de cette acidification et d'abaisser légèrement le pH atteint, pratiquement sans poser de problème de postacidification.

Dans le cadre de chacune de ces deux formes d'exécution préférées, il est possible de maintenir un relativement grand nombre de cellules vivantes de chacun des microorganismes dans les yogourts obtenus.

Pour mettre en oeuvre le procédé de préparation d'un yogourt selon la présente invention, on peut utiliser comme matière première un lait animal et/ou végétal, frais ou reconstitué, écrémé, semi-écrémé ou entier, pasteurisé, comprenant 10-20% en poids de matière sèche. On inocule de préférence ce lait avec 0,2-5%, de préférence 0,5-3%, en volume d'une culture contenant 10⁶-10¹⁰, de préférence 10⁸-10⁹, cellules/ml dudit mutant de Lactobacillus bulgaricus et 1-5%, de préférence 2-4%, en volume d'une culture contenant 10⁶-10¹⁰, de préférence 10⁸-10⁹, cellules/ml de Streptococcus thermophilus.

On peut faire fermenter le lait durant 2,5-15 h à 35-48°C. Le pH atteint au cours de cette fermentation ou acidification peut varier entre environ 4,3 et 4,8 dans le cadre de ladite première forme d'exécution, à savoir celle où ledit fragment manquant d'ADN de L.bulgaricus ne comprend qu'une partie au moins du gène bêta-galactosidase. Ce pH peut être abaissé de environ 0,1-0,5 unités par addition de environ 0,2-2% en poids de glucose au lait de départ.

Le pH atteint au cours de cette fermentation ou acidification peut varier entre environ 4,5 et 5,0 dans le cadre de ladite deuxième forme d'exécution, à savoir celle ou ledit fragment manquant d'ADN de L.bulgaricus comprend une partie au moins du gène bêta-galactosidase et s'étend au moins en aval de ce gène sur au moins 1,0 kb. Ce pH peut être abaissé de environ 0,05-0,3 unités par addition de environ 0,2-2% en poids de glucose au lait de départ.

On peut obtenir ainsi un yogourt contenant des nombres respectifs de cellules/ml dudit mutant de L.bulgaricus et de S.thermophilus comparables aux nombres de cellules/ml présentés par les cultures utilisées pour l'incubation. Les rapports entre ces nombres respectifs sont situés de préférence entre environ 1:20 et 1:7, voire entre 1:20 et 1:4 dans le cadre de ladite première forme d'exécution, et entre environ 1:10 et 1:4, voire entre 1:10 et 1:1 dans le cadre de ladite deuxième forme d'exécution.

Ce yogourt présente donc des qualités organoleptiques présentant le caractère typique du yogourt traditionnel dû à l'utilisation en symbiose de L.bulgaricus et S.thermophilus. Il peut être conservé durant plusieurs semaines sous réfrigération, voire à température ambiante pour ladite deuxième forme d'exécution, sans que le pH chute de plus de environ 0,05-0,5 unités, sans qu'apparaisse de goût amer, et sans que le nombre de cellules vivantes qu'il contient ne présente de variations notables.

Pour mettre en oeuvre le procédé de sélection d'un mutant de L.bulgaricus selon la présente invention, on choisit de préférence comme souche mère une souche utilisée commercialement et présentant donc toutes les qualités requises pour la production d'un bon yogourt traditionnel du commerce.

Avec une telle souche mère, on peut réaliser un criblage sur plaque X-gal tel que décrit par B.Mollet et al., Journal of Bacteriology 172, 5670-5676 (1990) en référence à J.H.Miller (1972), par exemple. On peut isoler ainsi des colonies présentant une mutation bêta-galactosidase moins, autrement dit des mutants incapables de fermenter le lactose du fait d'un disfonctionnement de l'enzyme bêta-galactosidase grâce à laquelle L.bulgaricus doit normalement hydrolyser le lactose en glucose et galactose avant de pouvoir consommer le glucose.

On peut soumettre ces colonies de mutants à un test de stabilité en les cultivant dans un milieu contenant du lactose comme seule source d'énergie, tel qu'un lait de vache, par exemple, afin d'éliminer les mutants susceptibles de retourner spontanément à l'état original bêta-galactosidase plus.

On peut extraire l'ADN chromosomique des mutants stables de la manière décrite par M.Delley et al., Appl. Environ. Microbiol. 56, 1967-70 (1990), par exemple. On peut digérer l'ADN à l'aide d'enzymes de restriction telles que BamHI, EcoRI, HindIII, SalI et TaqI, par exemple.

On soumet alors l'ADN digéré à un test de détection de fragments compris dans une région comprenant le gène bêta-galactosidase. Pour ce faire, on peut séparer les fragments d'ADN ainsi obtenus sur gel d'agarose, les placer sur une membrane de transfert pour criblage de gènes par hybridation, et réaliser une hybridation avec une sonde ADN portant le gène bêta-galactosidase selon la méthode des taches de Southern (E.Southern, J.Mol.Biol. 98, 503-517, 1975), par exemple. On peut utiliser comme sonde ADN un fragment SalI-BamHI du plasmide pMZ2 (B.Mollet et al., J.Bacteriol.172, 5670-5676, 1990), fragment que l'on peut marquer au ³²P, qui présente une longueur de 4,3 kb et qui contient tout le gène bêta-galactosidase, par exemple.

Pour réaliser ledit test de détection, on peut également appliquer la méthode de la PCR avec des amorces spécifiques, par exemple (PCR est le sigle de l'expression anglaise "Polymerase Chain Reaction"; cf. Saiki et al., Science 230, 1350-1354, 1985 et Saiki et al., Science 239, 487-491, 1988).

On peut sélectionner alors un mutant présentant un ADN dans lequel manque un fragment comprenant une partie au moins du gène bêta-galactosidase. On a constaté en effet que de tels mutants d'une souche mère commerciale pouvaient être utilisés avantageusement en symbiose avec S.thermophilus pour la préparation des présents yogourts.

On a constaté en particulier que de tels mutants présentant un ADN dans lequel manque un fragment ne comprenant qu'une partie du gène bêta-galactosidase, fragment qui peut présenter une longueur comprise entre quelques paires de base et la quasi totalité de la longueur du gène bêta-galactosidase, se prêtent particulièrement bien à la préparation d'un yogourt à longue conservation sous réfrigération dans des pays à climat tempéré, voire relativement froid. Parmi divers mutants sélectionnés de ce type, l'un a été déposé, à titre d'exemple, selon le Traité de Budapest, le 29.03.91., à la Collection Nationale de Cultures de Microorganismes (CNCM), Institut Pasteur, 28 rue du Dr Roux, 7524 Paris Cedex 15, France, où il a reçu le No CNCM I-1068.

Des détails concernant la morphologie et les propriétés de cette souche CNCM I-1068 sont donnés ci-après:

### Morphologie:

- Microorganisme Gram positif, catalase négatif et anaérobe facultatif.
- Bacilles droits, sans flagelles, ni formation de spores.

### Fermentation des sucres:

- Production d'acide à partir de:
   - D-glucose
   - D-fructose
   - D-mannose

### Autres:

- Pas de production d'acide à partir du lactose (pas d'enzyme bêta-galactosidase fonctionnelle).

On a constaté aussi en particulier que de tels mutants présentant un ADN dans lequel manque un fragment comprenant une partie au moins du gène bêta-galactosidase et s'étendant au moins en aval de ce gène sur au moins 1,0 kb, se prêtent particulièrement bien à la préparation d'un yogourt à longue conservation sous réfrigération dans des pays à climat relativement chaud. Parmi les divers mutants sélectionnés de ce type, l'un a été déposé, à titre d'exemple, selon le Traité de Budapest, le 29.03.91, à la Collection Nationale de Cultures de Microorganismes (CNCM), Institut Pasteur, 28 rue du Dr Roux, 7524 Paris Cedex 15, France, où il a reçu le No CNCM I-1067.

Des détails concernant la morphologie et les propriétés de cette souche CNCM I-1067 sont donnés ci-après:

### Morphologie:

- Microorganisme Gram positif, catalase négatif et anaérobe facultatif.
- Bacilles droits, sans flagelles, ni formation de spores.

### Fermentation des sucres:

- Production d'acide à partir de:
   - D-glucose
   - D-fructose
   - D-mannose

### Autres:

- Pas de production d'acide à partir du lactose (pas d'enzyme bêta-galactosidase fonctionnelle).

Les exemples ci-après sont présentés à titre d'illustration des produits et procédés selon la présente invention. Les pourcentages y sont donnés en poids, sauf indication contraire.

### Exemple 1

On stérilise 5 litres de milieu de culture MRS à 121°C durant 15 min. On les inocule avec 5% en volume d'une culture active de la souche Lactobacillus bulgaricus CNCM I-1068 contenant environ 10⁹ cellules/ml. On incube à 41°C durant 8 h et l'on obtient un levain contenant 4,5-10⁸ cellules/ml.

On stérilise à 121°C durant 15 min 5 litres de lait écrémé reconstitué à 10% de matière sèche auquel on a ajouté 0,1% d'extrait de levure. On les inocule avec 2% d'une culture active de Streptococcus thermophilus épaississant du commerce contenant environ 10⁹ cellules/ml. On incube à 41°C durant 4 h et l'on obtient un levain contenant 4,5.10⁸ cellules/ml.

On inocule ensuite un lot de lait entier à 3,7% de matière grasse renforcé avec 2,5% de lait écrémé en poudre et pasteurisé 30 min à 90°C, avec 2% en volume du levain de L.bulgaricus I-1068 et 3% en volume du levain de S.thermophilus épaississant. On brasse le lait inoculé, on le répartit en pots et on l'incube durant 4 h à 41°C.

On obtient un yogourt qui présente une bonne texture ferme et lisse, ainsi qu'une flaveur douce agréable, typique de ce genre de produit. Ce yogourt a un pH de 4,53 et contient 4,6.10⁷ cellules/ml de L.bulgaricus I-1068 et 6,8.10⁸ cellules/ml de S.thermophilus.

On soumet ce yogourt à un test de conservation à 4°C et à 12°C. Dans ce test, on mesure le pH et on déguste le produit après 1, 7, 14 et 24 d (jours) de conservation. Les résultats sont présentés dans le tableau 1 ci-après.

### Exemple 2

On procède de la manière décrite à l'exemple 1, mais on utilise la souche de L.bulgaricus CNCM I-1067 à la place de la souche CNCM I-1068.

Le levain de L.bulgaricus obtenu contient 5,2.10⁸ cellules/ml.

On obtient un yogourt présentant un pH de 4,55 après 8h30 d'incubation à 41°C. Il contient 5,6.10⁷ cellules/ml de L.bulgaricus CNCM I-1067 et 5,5.10⁸ cellules/ml de S.thermophilus. Il présente une bonne texture et une flaveur agréable comparables à celles du yogourt obtenu à l'exemple 1.

On soumet ce yogourt au test de conservation décrit à l'exemple 1. Les résultats sont présentés dans le tableau 1 ci-après.

### Exemple comparatif i)

A titre de comparaison, on procède de la manière décrite à l'exemple 1 en remplaçant les deux mutants I-1067 et I-1068 par leur souche mère qui est une souche utilisée industriellement pour la fabrication de yogourts. On obtient un yogourt témoin (i) présentant un pH de 4,53 après 3h30 d'incubation à 41°C. Il contient 2,9.10⁷ cellules/ml de L.bulgaricus et 4,9.10⁸ cellules/ml de S.thermophilus. Il présente une bonne texture et une flaveur douce agréable.

On soumet ce yogourt témoin (i) au test de conservation décrit à l'exemple 1. Les résultats sont présentés dans le tableau 1 ci-après.

**Tableau 1**

| Ex no (CNCM no) | Temps de stockage (d) | Conservation à 4°C | | Conservation à 12°C | |
|---|---|---|---|---|---|
| | | pH | dégustation | pH | dégustation |
| 1 (I-1068) | 1 | 4,53 | bon, doux | 4,53 | bon, doux |
| | 7 | 4,41 | bon, doux | 4,20 | bon, aromatique |
| | 14 | 4,30 | bon, aromatique | 4,13 | bon, aromatique |
| | 24 | 4,22 | bon, aromatique | 4,06 | bon, aromatique |
| 2 (I-1067) | 1 | 4,55 | bon, doux | 4,55 | bon, doux |
| | 7 | 4,48 | bon, doux | 4,35 | bon, aromatique |
| | 14 | 4,46 | doux, aromatique | 4,22 | bon, aromatique |
| | 24 | 4,42 | doux, aromatique | 4,20 | bon, aromatique |
| comparatif i) (témoin i) | 1 | 4,53 | bon, doux | 4,53 | bon, doux |
| | 7 | 4,27 | lég.acide | 4,18 | acide |
| | 14 | 4,18 | lég.amer | 3,97 | amer |
| | 24 | 4,16 | lég.amer | 3,92 | très acide, amer |

Ces résultats montrent que les deux yogourts des exemples 1 et 2 sont très stables à la conservation, leur postacidification étant beaucoup moins rapide que celle du yogourt témoin. La postacidification du yogourt de l'exemple 2 est encore plus faible que celle du yogourt de l'exemple 1. Enfin, contrairement à ce qui se passe avec le yogourt témoin, on ne constate l'apparition d'aucun goût amer, même léger, dans les yogourts des exemples 1 et 2.

### Exemples 3-6

On procède de la même manière qu'à l'exemple 1, à l'exception du fait que l'on ajoute 0,5% de glucose dans le lait à l'exemple 3 et 1% de glucose dans le lait à l'exemple 4 avant de le pasteuriser.

De même, on procède comme à l'exemple 2, à l'exception du fait que l'on ajoute 0,5% de glucose dans le lait à l'exemple 5 et 1% de glucose dans le lait à l'exemple 6.

Le lait inoculé est incubé à 41°C, pendant 3h40 jusqu'à un pH de 4,55 à l'exemple 3, pendant 3h40 jusqu'à un pH de 4,53 à l'exemple 4, et pendant 8 h jusqu'à un pH de 4,63 aux exemples 5 et 6.

Les yogourts ainsi obtenus présentent une bonne texture et une flaveur agréable. Leurs teneurs respectives en L.bulgaricus et S.thermophilus, exprimées en nombre de cellules actives par ml, sont de 4,7.10⁷ (I-1068) et 6,4.10⁸, 7.10⁷ (I-1068) et 9,0.10⁸, 7,8.10⁷ (I-1067) et 7,5.10⁸, ainsi que 7,9.10⁷ (I-1067) et 5,7.10⁸ pour les exemples respectifs 4, 5, 6 et 7.

On soumet tous ces yogourts au test de conservation décrit à l'exemple 1 et l'on obtient les résultats présentés au tableau 2 ci-après:

**Tableau 2**

| Ex no (CNCM no) | Temps de stockage (d) | Conservation à 4°C | | Conservation à 12°C | |
|---|---|---|---|---|---|
| | | pH | dégustation | pH | dégustation |
| 3 (I-1068) | 1 | 4,55 | bon, doux | 4,55 | bon, doux |
| | 7 | 4,36 | bon, aromatique | 4,07 | bon, acide |
| | 14 | 4,30 | bon, aromatique | 4,01 | bon, acide |
| | 24 | 4,25 | bon, aromatique | 3,94 | bon, acide |
| 4 (I-1068) | 1 | 4,53 | bon, doux | 4,53 | bon, doux |
| | 7 | 4,24 | bon, aromatique | 4,00 | bon, acide |
| | 14 | 4,15 | bon, aromatique | 3,93 | très acide |
| | 24 | 4,08 | bon, aromatique | 3,84 | très acide |
| 5 (I-1067) | 1 | 4,63 | bon, doux | 4,63 | bon, doux |
| | 7 | 4,42 | bon, aromatique | 4,30 | aromatique |
| | 14 | 4,42 | bon, aromatique | 4,19 | aromatique |
| | 24 | 4,40 | bon, aromatique | 4,16 | aromatique |
| 6 (I-1067) | 1 | 4,63 | bon, doux | 4,63 | bon, doux |
| | 7 | 4,42 | bon, aromatique | 4,31 | aromatique |
| | 14 | 4,42 | bon, aromatique | 4,22 | aromatique |
| | 24 | 4,40 | bon, aromatique | 4,15 | aromatique |

Ces résultats montrent que l'on peut moduler le degré de postacidification du yogourt en ajoutant du glucose dans le lait lorsque le mutant de L.bulgaricus utilisé est du type dans lequel ledit fragment d'ADN manquant ne comprend qu'une partie du gène bêta-galactosidase (Exemples 3 et 4, L.bulgaricus CNCM I-1068). Cette postacidification plus ou moins réduite fait qu'un tel mutant est plus particulièrement destiné à être utilisé pour préparer des yogourts dans des pays à climat tempéré ou relativement froid.

Par contre, on ne modifie qu'à peine le degré de postacidification du yogourt en ajoutant du glucose dans le lait lorsque le mutant de L.bulgaricus utilisé est du type dans lequel ledit fragment d'ADN manquant comprend une partie au moins du gène bêta-galactosidase et s'étend au moins en aval de ce gène sur au moins 1,0 kb (Exemples 5 et 6, L.bulgaricus CNCM I-1067). Cette absence d'influence du glucose sur la postacidification permet l'utilisation d'un mutant de ce type pour la préparation de yogourts aromatisés sucrés, par exemple. La postacidification elle-même très réduite, même à 12°C, destine en outre un tel mutant à être plus particulièrement utilisé pour préparer des yogourts dans des pays à climat relativement chaud.

### Exemple 7

On prépare deux levains semblables à ceux de l'exemple 2, l'un contenant 4,3.10⁸ cellules/ml de L.bulgaricus CNCM I-1067 et l'autre contenant 6.10⁸ cellules/ml de S.thermophilus filant.

Dans un lait partiellement écrémé contenant 2,8% de matière grasse renforcé avec 5% de lait écrémé en poudre, et pasteurisé 30 minutes à 90°C, on inocule 2% en volume du levain de L.bulgaricus et 3,5% en volume du levain de S.thermophilus épaississant. Après incubation pendant 8 heures à 41°C, on refroidit et on brasse le yogourt obtenu, avant de le répartir en pots. On obtient un yogourt de type brassé présentant un pH de 4,62, une bonne texture crémeuse et une flaveur agréable, qui contient 6,4.10⁷ cellules/ml de L.bulgaricus CNCM I-1067 et 4,5.10⁸ cellules/ml de S.thermophilus filant.

On soumet ce yogourt au test de conservation décrit à l'exemple 1. Les résultats du test sont présentés au tableau 3 ci-après.

### Exemple comparatif ii)

On procède de la manière décrite à l'exemple 7, à l'exception du fait que l'on prépare un levain de L.bulgaricus non pas avec la souche CNCM I-1067, mais avec l'une des souches présentant une faible activité protéolytique du brevet US 4425366.

On inocule ensuite un même lait et dans les mêmes conditions qu'à l'exemple 7. Après 8h30 d'incubation à 41°C, on obtient également un yogourt de type brassé présentant un pH de 4,63, une texture crémeuse et une flaveur agréable, qui contient 1,6.10⁷ cellules/ml de L.bulgaricus et 2,6.10⁸ cellules/ml de S.thermophilus.

On soumet ce yogourt témoin au test de conservation décrit à l'exemple 1 et l'on obtient les résultats présentés au tableau 3 ci-après.

**Tableau 3**

| Ex no (CNCM no) | Temps de stockage (d) | Conservation à 4°C | | Conservation à 12°C | |
|---|---|---|---|---|---|
| | | pH | dégustation | pH | dégustation |
| 7 (I-1067) | 1 | 4,62 | bon, doux | 4,62 | bon, doux |
| | 7 | 4,53 | bon, doux | 4,46 | aromatique |
| | 14 | 4,48 | lég. aromatique | 4,29 | aromatique |
| | 24 | 4,47 | lég. aromatique | 4,24 | aromatique |
| comparatif ii) (témoin ii) | 1 | 4,63 | doux | 4,63 | bon, doux |
| | 7 | 4,54 | doux | 4,39 | bon, doux |
| | 14 | 4,47 | doux | 4,25 | lég. acide |
| | 24 | 4,39 | lég. aromatique | 4,19 | lég. acide |

Ces résultats montrent que la souche CNCM I-1067 peut avantageusement remplacer l'une des souches de US 4425366 dans la préparation d'un yogourt longue conservation, car, pour une postacidification comparable, elle permet d'obtenir un yogourt plus aromatique qui contient un nombre relatif de cellules de L.bulgaricus plus élevé.

### Exemples 8 - 11 (sélection)

De la manière décrite plus haut, on met en oeuvre le procédé de sélection d'un mutant de L.bulgaricus selon la présente invention à partir de quatre souches mères différentes utilisées commercialement, dont deux présentent des propriétés texturantes, à savoir filantes. On sélectionne ainsi divers mutants présentant un ADN dans lequel manque un fragment ne comprenant qu'une partie du gène bêta-galactosidase.

Parmi ces mutants, quatre, à savoir un pour chaque souche mère différente, ont été déposés à titre d'exemple, selon le Traité de Budapest, le 02.04.92, à la Collection Nationale de Cultures de Microorganismes (CNCM), Institut Pasteur, 28 rue du Dr Roux, 7524 Paris Cedex 15, France, où ils ont reçu les Nos CNCM I-1191, I-1193, I-1196 et I-1198.

Des détails concernant la morphologie et les propriétés de ces souches sont donnés ci-après :

### Morphologie :

- Microorganismes Gram positifs, catalase négatifs, anaérobes facultatifs.
- Bacilles droits, sans flagelles, pas de formation de spores.

### Fermentation des sucres :

- Production d'acide à partir de :

| | I-1191 | I-1193 | I-1196 | I-1198 |
|---|---|---|---|---|
| D-glucose | + | + | + | + |
| D-fructose | + | + | + | + |
| D-mannose | + | + | + | + |
| Trehalose | + | - | - | - |
| | | | | |

### Autres :

- Pas de production d'acide à partir du lactose (pas d'enzyme bêta-galactosidase fonctionnelle).
- Les souches CNCM I-1196 et I-1198 présentent des propriétés texturantes (production de polysaccharides).

### Exemples 8 et 9 (préparation de yogourts)

On prépare deux levains contenant chacun environ 10⁸ cellules/ml de l'une des souches de L.bulgaricus CNCM I-1191 et I-1193, ainsi qu'un levain contenant environ 10⁹ cellules/ml d'une culture de Streptococcus thermophilus du commerce.

On prépare deux lots de 40 l de lait frais à 3,7% de matière grasse additionné de 1,5% de lait écrémé en poudre et pasteurisé 3 min à 98°C. On inocule chacun des deux lots avec 2% en volume de l'un des deux levains de L.bulgaricus CNCM I-1191 et I-1193, et l'on incube à 40°C durant respectivement 9 h et 4 h 15 min.

On obtient des yogourts qui présentent une texture ferme et lisse, une flaveur douce et typique, un pH de 4,65 et des teneurs respectives en nombre de cellules de L.bulgaricus et de S.thermophilus par ml de 4,5.10⁶ (I-1191) et 2,2.10⁸, ainsi que 2,4.10⁷ (I-1193) et 5,5.10⁸.

On soumet ces yogourts au test de conservation décrit à l'exemple 1. Les résultats sont présentés dans le tableau 4 ci-après.

### Exemples 10 et 11 (préparation de yogourts)

On prépare deux levains contenant chacun environ 10⁸ cellules/ml de l'une des souches de L.bulgaricus filantes CNCM I-1196 et I-1198, ainsi qu'un levain contenant environ 10⁹ cellules/ml d'une culture de S.thermophilus du commerce.

On prépare deux lots de 20 l de lait à 1,5% de matière grasse additionné de 2,5% de lait écrémé en poudre et pasteurisé. On inocule chacun des deux lots avec 2% en volume de l'un des deux levains de L.bulgaricus filant CNCM I-1196 et I-1198, et l'on incube 5 h à 40°C.

On obtient des yogourts qui présentent une texture crémeuse, une flaveur douce et typique, un pH de 4,65 et des teneurs respectives en nombre de cellules de L.bulgaricus et de S.thermophilus par ml de 6,0.10⁶ (I-1196) et 3,5.10⁸, ainsi que 1,8.10⁷ (I-1198) et 5,0.10⁷.

On soumet ces yogourts au test de conservation décrit à l'exemple 1. Les résultats sont présentés dans le tableau 4 ci-après.

**Tableau 4**

| Ex no (CNCM no) | Temps de stockage (d) | Conservation à 4°C | | Conservation à 12°C | |
|---|---|---|---|---|---|
| | | pH | dégustation | pH | dégustation |
| 8 (I-1191) | 1 | 4,50 | bon, doux | 4,50 | bon, doux |
| | 7 | 4,39 | bon, doux | 4,25 | bon, aromatique |
| | 14 | 4,33 | bon, aromatique | 4,18 | bon, aromatique |
| | 24 | 4,28 | bon, aromatique | 4,05 | bon, aromatique |
| 9 (I-1193) | 1 | 4,44 | bon, doux | 4,44 | bon, doux |
| | 7 | 4,32 | bon, aromatique | 4,20 | bon, aromatique |
| | 14 | 4,25 | bon, aromatique | 4,12 | bon, aromatique |
| | 24 | 4,23 | bon, aromatique | 4,02 | bon, aromatique |
| 10 (I-1196) | 1 | 4,45 | bon, doux | 4,45 | bon, doux |
| | 7 | 4,43 | bon, doux | 4,32 | bon, aromatique |
| | 14 | 4,41 | bon, doux | 4,23 | bon, aromatique |
| | 24 | 4,34 | bon, doux | 4,23 | bon, aromatique |
| 11 (I-1198) | 1 | 4,50 | bon, doux | 4,50 | bon, doux |
| | 7 | 4,38 | bon, doux | 4,29 | bon, aromatique |
| | 14 | 4,40 | bon, doux | 4,24 | bon, aromatique |
| | 24 | 4,35 | doux, aromatique | 4,20 | bon, aromatique |

Si l'on compare ces résultats entre eux ainsi qu'avec ceux illustrés au Tableau 1, Ex. no 1, on constate que la postacidification de tous ces yogourts est très semblable et beaucoup moins rapide que celle du yogourt témoin (Tableau 1, Ex. comparatif i). Ceci montre que la mise en oeuvre du procédé de sélection selon la présente invention permet de sélectionner à partir de souches mères différentes, autrement dit à partir de n'importe quelle souche de L.bulgaricus du commerce, des mutants, notamment des mutants présentant un ADN dans lequel manque un fragment ne contenant qu'une partie du gène bêta-galactosidase, qui, utilisés en combinaison avec n'importe quelle souche de S.thermophilus du commerce permettent de préparer des yogourts de caractère traditionnel présentant pratiquement les mêmes propriétés remarquables de conservation.

### Exemples 12 - 15 (sélection)

De la manière décrite plus haut, on met en oeuvre le procédé de sélection d'un mutant de L.bulgaricus selon la présente invention à partir de quatre souches mère différentes utilisées commercialement, dont deux présentent des propriétés texturantes, à savoir filantes. On sélectionne ainsi divers mutants présentant un ADN dans lequel manque un fragment comprenant un partie au moins du gène bêta-galactosidase et s'étendant au moins en aval de ce gène sur au moins 1,0 kb.

Parmi ces mutants, quatre, à savoir un pour chaque souche mère différente, ont été déposés à titre d'exemple, selon le Traité de Budapest, le 02.04.92, à la Collection Nationale de Cultures de Microorganismes (CNCM), Institut Pasteur, 28 rue du Dr. Roux, 7524 Paris Cedex 15, France, où ils ont reçu les Nos CNCM I-1192, I-1194, I-1195 et I-1197.

Des détails concernant la morphologie et les propriétés de ces souches sont donnés ci-après :

### Morphologie :

- Microorganismes Gram positifs, catalase négatifs, anaérobes facultatifs.
- Bacilles droits, sans flagelles, pas de formation de spores.

### Fermentation des sucres :

- Production d'acide à partir de :

| | I-1192 | I-1194 | I-1195 | I-1197 |
|---|---|---|---|---|
| D-glucose | + | + | + | + |
| D-fructose | + | + | + | + |
| D-mannose | + | + | + | + |
| Trehalose | + | - | - | - |

### Autres :

- Pas de production d'acide à partir du lactose (pas d'enzyme bêta-galactosidase fonctionnelle).
- Les souches CNCM I-1195 et I-1197 présentent des propriétés texturantes (production de polysaccharides).

### Exemples 12 - 15 (préparation de yogourts)

On prépare quatre levains contenant chacun environ 10⁸ cellules/ml de l'une des souches de L.bulgaricus CNCM I-1192, I-1194, I-1195 et I-1197, ainsi qu'un levain contenant environ cellules/ml d'une culture de Streptococcus thermophilus du commerce.

On prépare quatre lots de 40 l de lait frais à 3,7% de matière grasse additionné de 1,5% de lait écrémé en poudre et pasteurisé 3 min à 98°C. On inocule chacun des quatre lots avec 2% en volume de l'un des quatre levains de L.bulgaricus CNCM I-1192, I-1194, I-1195 et I-1197, et l'on incube 9 h à 40°C.

On obtient des yogourts qui présentent une texture ferme et lisse (I-1192, I-1194) ou crémeuse (I-1194, I-1195), une flaveur douce à très douce et typique, des pH respectifs de 4,60 (I-1192), 4,80 (I-1194), 4,76 (I-1195) et 4,66 (I-1197), et des teneurs respectives en nombre de cellules de L.bulgaricus et de S.thermophilus par ml de 9,9.10⁶ (I-1192) et 1,1.10⁸, 3,5.10⁶ (I-1194) et 7,9.10⁷, 2,3.10⁶ (I-1195) et 9,3.10⁷, ainsi que 1,7.10⁷ (I-1197) et 1,7.10⁸.

On soumet ces yogourts au test de conservation décrit à l'exemple 1. Les résultats sont présentés dans le tableau 5 ci-après.

**Tableau 5**

| Ex no (CNCM no) | Temps de stockage (d) | Conservation à 4°C | | Conservation à 12°C | |
|---|---|---|---|---|---|
| | | pH | dégustation | pH | dégustation |
| 12 (I-1192) | 1 | 4,57 | bon, doux | 4,57 | bon, doux |
| | 7 | 4,46 | bon, doux | 4,33 | bon, aromatique |
| | 14 | 4,49 | bon, doux | 4,33 | bon, aromatique |
| | 24 | 4,40 | bon, aromatique | 4,26 | bon, aromatique |
| 13 (I-1194) | 1 | 4,90 | très doux | 4,90 | très doux |
| | 7 | 4,77 | très doux | 4,60 | bon, doux |
| | 14 | 4,74 | très doux | 4,64 | bon, doux |
| | 24 | 4,66 | bon, doux | 4,53 | bon, doux |
| 14 (I-1195) | 1 | 4,61 | bon, doux | 4,61 | bon, doux |
| | 7 | 4,57 | bon, doux | 4,43 | bon, doux |
| | 14 | 4,59 | bon, doux | 4,34 | bon, aromatique |
| | 24 | 4,49 | bon, doux | 4,29 | bon, aromatique |
| 15 (I-1196) | 1 | 4,52 | bon, doux | 4,52 | bon, doux |
| | 7 | 4,48 | bon, doux | 4,34 | bon, aromatique |
| | 14 | 4,41 | bon, doux | 4,18 | bon, aromatique |
| | 24 | 4,31 | doux, aromatique | 4,20 | bon, aromatique |

Si l'on compare ces résultats entre eux ainsi qu'avec ceux illustrés au Tableau 1, Ex. no 2, on constate que la postacidification de tous ces yogourts est très semblable, beaucoup moins rapide que celle du yogourt témoin (Tableau 1, Ex. comparatif i), et même encore plus faible que celle des yogourts des exemples 1,8,9,10 et 11. Ceci montre que la mise en oeuvre du procédé de sélection selon la présente invention permet de sélectionner à partir de souches mère différentes, autrement dit à partir de n'importe quelle souche de L.bulgaricus du commerce, des mutants, notamment des mutants présentant un ADN dans lequel manque un fragment contenant une partie au moins du gène bêta-galactosidase et s'étendant au moins en aval de ce gène sur au moins 1,0 kb, qui, utilisés en combinaison avec n'importe quelle souche de S.thermophilus du commerce permettent de préparer des yogourts de caractère traditionnel présentant pratiquement les mêmes propriétés remarquables de conservation.

## Revendications

1. Yogourt comprenant
- 10-20% en poids de matière sèche d'un lait animal et/ou végétal,
- de 10⁶-10¹⁰ cellules/ml d'un mutant de Lactobacillus bulgaricus dans l'ADN duquel manque un fragment comprenant une partie au moins du gène bêta-galactosidase, et
- de 10⁶-10¹⁰ cellules/ml de Streptococcus thermophilus.

2. Yogourt selon la revendication 1, dans lequel ledit fragment d'ADN manquant audit mutant de L.bulgaricus ne comprend qu'une partie du gène bêta-galactosidase.

3. Yogourt selon la revendication 1, dans lequel ledit fragment d'ADN manquant audit mutant de L.bulgaricus comprend une partie au moins du gène bêta-galactosidase et s'étend au moins en aval de ce gène sur au moins 1,0 kb.

4. Yogourt selon la revendication 1, dans lequel ledit mutant est l'une des souches de Lactobacillus bulgaricus CNCM I-1067, I-1068, I-1191, I-1192, I-1193, I-1194, I-1195, I-1196, I-1197 et I-1198.

5. Procédé de préparation d'un yogourt, dans lequel on fait fermenter un lait avec une combinaison d'au moins une souche de Streptococcus thermophilus et d'un mutant de Lactobacillus bulgaricus dans l'ADN duquel manque un fragment comprenant une partie au moins du gène bêta-galactosidase.

6. Procédé selon la revendication 5, dans lequel on inocule ledit lait avec 0,2-5% en volume d'une culture contenant 10⁶-10¹⁰ cellules/ml dudit mutant de Lactobacillus bulgaricus et 1-5% en volume d'une culture contenant 10⁶-10¹⁰ cellules/ml de Streptococcus thermophilus.

7. Procédé selon la revendication 5, dans lequel on fait fermenter un lait animal et/ou végétal comprenant 10-20% en poids de matière sèche, durant 2,5-15 h à 35-48°C.

8. Procédé selon la revendication 5, dans lequel ledit mutant de L.bulgaricus présente un ADN où manque un fragment ne comprenant qu'une partie du gène bêta-galactosidase.

9. Procédé selon la revendication 5, dans lequel ledit mutant de L.bulgaricus présente un ADN où manque un fragment comprenant une partie au moins du gène bêta-galactosidase et s'étendant au moins en aval de ce gène sur au moins 1,0 kb.

10. Procédé selon la revendication 5, dans lequel ledit mutant est l'une des souches de Lactobacillus bulgaricus CNCM I-1067, I-1068, I-1191, I-1192, I-1193, I-1194, I-1195, I-1196, I-1197 et I-1198.

## Claims

1. A yogurt containing
- 10 to 20% by weight dry matter of an animal and/or vegetable milk,
- 10⁶-10¹⁰ cells/ml of a mutant of Lactobacillus bulgaricus in the DNA of which a fragment containing at least part of the β-galactosidase gene is missing and
- 10⁶-10¹⁰ cells/ml of Streptococcus thermophilus.

2. A yogurt as claimed in claim, in which the DNA fragment from which the L. bulgaricus mutant is missing contains only part of the β-galactosidase gene.

3. A yogurt as claimed in claim 1, in which the DNA fragment from which the L. bulgaricus mutant is missing contains at least part of the β-galactosidase gene and extends at least below that gene over at least 1.0 kb.

4. A yogurt as claimed in claim 1, in which the mutant is one of the Lactobacillus bulgaricus strains CNCM I-1067, I-1068, I-1191, I-1192, I-1193, I-1194, I-1195, I-1196, I-1197 and I-1198.

5. A process for the production of a yogurt, in which a milk is fermented with a combination of at least one strain of Streptococcus thermophilus and a mutant of Lactobacillus bulgaricus in the DNA of which a fragment containing at least part of the β-galactosidase gene is missing.

6. A process as claimed in claim 5, in which the milk is inoculated with 0.2 to 5% by volume of a culture containing 10⁶-10¹⁰ cells/ml of the Lactobacillus bulgaricus mutant and 1 to 5% by volume of a culture containing 10⁶-10¹⁰ cells/ml of Streptococcus thermophilus.

7. A process as claimed in claim 5, in which an animal and/or vegetable milk containing 10 to 20% dry matter is fermented for 2.5 to 15 h at 35 to 48°C.

8. A process as claimed in claim 5, in which the L. bulgaricus mutant has a DNA from which a fragment containing only part of the β-galactosidase gene is missing.

9. A process as claimed in claim 5, in which the L. bulgaricus mutant has a DNA in which a fragment containing at least part of the β-galactosidase gene and extending at least below that gene over at least 1.0 kb is missing.

10. A process as claimed in claim 5, in which the mutant is one of the Lactobacillus bulgaricus strains CNCM I-1067, I-1068, I-1191, I-1192, I-1193, I-1194, I-1195, I-1196, I-1197 and I-1198.

## Patentansprüche

1. Joghurt, der aufweist
- 10-20 Gew.-% Trockenmasse einer Tier- und/oder Pflanzenmilch,
- von 10⁶-10¹⁰ Zellen/ml einer Mutante von Lactobacillus bulgaricus, in deren DNA ein Fragment fehlt, das wenigstens einen Teil des β-Galactosidasegens umfaßt, und
- von 10⁶-10¹⁰ Zellen/ml Streptococcus thermophilus.

2. Joghurt nach Anspruch 1, bei dem das bei der L.bulgaricus-Mutante fehlende DNA-Fragment nur einen Teil des β-Galactosidasegens umfaßt.

3. Joghurt nach Anspruch 1, bei dem das bei der L.bulgaricus-Mutante fehlende DNA-Fragment wenigstens einen Teil des β-Galactosidasegens umfaßt und sich wenigstens stromab von dem genannten Gen über wenigstens 1,0 kb erstreckt.

4. Joghurt nach Anspruch 1, bei dem die genannte Mutante einer der Lactobacillus bulgaricus-Stämme CNCM I-1067, I-1068, I-1191, I-1192, I-1193, I-1194, I-1195, I-1196, I-1197 und I-1198 ist.

5. Verfahren zur Herstellung eines Joghurts, bei dem man eine Milch mit einer Kombination aus wenigstens einem Streptococcus thermophilus-Stamm und einer Lactobacillus bulgaricus-Mutante fermentiert, in deren DNA ein Fragment fehlt, das wenigstens einen Teil des β-Galactosidasegens umfaßt.

6. Verfahren nach Anspruch 5, bei der man die genannte Milch mit 0,2-5 Vol.-% einer Kultur beimpft, die 10⁶ - 10¹⁰ Zellen/ml der genannten Lactobacillus bulgaricus-Mutante enthält, und 1-5 Vol.-% einer Kultur, die 10⁶-10¹⁰ Zellen/ml Streptococcus thermophilus enthält.

7. Verfahren nach Anspruch 5, bei dem man eine Tier- und/- oder Pflanzenmilch fermentiert, die 10-20 Gew.-% Trockenmasse aufweist, und zwar während 2,5-15 h bei 35-48°C.

8. Verfahren nach Anspruch 5, bei dem die genannte L.bulgaricus-Mutante eine DNA aufweist, bei der ein Fragment fehlt, das nur einen Teil des β-Galactosidasegens umfaßt.

9. Verfahren nach Anspruch 5, bei dem die genannte L.bulgaricus-Mutante eine DNA aufweist, bei der ein Fragment fehlt, das wenigstens einen Teil des β-Galactosidasegens umfaßt und sich wenigstens stromab von dem genannten Gen über wenigstens 1,0 kb erstreckt.

10. Verfahren nach Anspruch 5, bei dem die genannte Mutante einer der Lactobacillus bulgaricus-Stämme CNCM I-1067, I-1068, I-1191, I-1192, I-1193, I-1194, I-1195, I-1196, I-1197 und I-1198 ist.
